# FASCICULE DE BREVET EUROPEEN

(11) **EP 0 840 629 B1**
(45) Date de publication et mention de la délivrance du brevet: **20.04.2005**
(21) Numéro de dépôt: 96924956.4
(22) Date de dépôt: 08.07.1996
(51) Int. Cl.: A61M 5/32, A61M 5/50

(54) **SERINGUE D'INJECTION AUTO-PROTEGEE**
INJEKTIONSSPRITZE MIT AUTOMATISCHEM NADELSCHUTZ
SELF-PROTECTED INJECTION SYRINGE

(30) Priorité: 12.07.1995 FR 9508462
(43) Date de publication de la demande: 13.05.1998
(60) Demande divisionnaire: 00111638.3
(73) Titulaire: SEDAT, 69540 Irigny (Rhône) (FR)
(72) Inventeur: PEROUSE, Eric, F-95290 L'Isle-Adam (FR)
(74) Mandataire: Moncheny, Michel
(86) Numéro de dépôt international: PCT/FR1996/001066
(87) Numéro de publication internationale: WO 1997/002855

(56) Documents cités:
- EP-A- 0 405 039
- EP-A- 0 467 173
- EP-A- 0 497 220
- WO-A-95/09022
- FR-A- 1 084 947
- FR-A- 2 700 959
- GB-A- 2 202 747
- US-A- 3 368 558
- US-A- 5 180 370
- US-A- 5 460 611

## Description

La présente invention concerne une seringue d'injection, du type comportant d'une part un corps de seringue allongé comprenant un tube et une paroi avant munie d'une aiguille d'injection, et d'autre part un piston arrière d'actionnement monté déplaçable à l'intérieur du tube.

On connaît des seringues du type précité, utilisées notamment pour effectuer des injections sous-cutanées ou intramusculaires dans le corps d'un patient.

Le développement des maladies transmises par voie sanguine a conduit au développement des seringues à usage unique, qui sont jetées après l'achèvement de l'injection. Ces seringues peuvent être livrées remplies de la substance à injecter, ou encore vides, la substance à injecter étant pompée au travers de l'aiguille d'injection, par aspiration obtenue par traction du piston arrière.

Les seringues connues présentent un danger pour l'opérateur immédiatement après le retrait de l'aiguille du corps du patient en fin d'injection. En effet, l'opérateur risque de se piquer avec la pointe de l'aiguille et d'être contaminé par les traces de sang résiduelles qu'elle porte.

Afin de limiter le danger présenté par la pointe de l'aiguille, il a été proposé des capuchons protecteurs que l'opérateur vient emboîter sur le corps de seringue pour recouvrir l'aiguille. Cependant, le capuchon devant être positionné manuellement, l'opérateur peut oublier d'installer celui-ci, ou encore être piqué par l'aiguille lors de sa mise en place. De plus, le capuchon ne peut être mis en place simultanément au retrait de l'aiguille du corps du patient, et l'aiguille reste ainsi non protégée pendant un court instant.

FR-A-2.700.959 décrit une seringue comportant un protecteur d'aiguille extérieur telle que définie dans le préambule de la revendication 1.

Dans la seringue antérieure, le protecteur d'aiguille est dissocié du piston d'actionnement de la seringue.

L'invention a pour but de fournir une seringue d'injection écartant tout risque de piqûre de l'opérateur par la pointe de l'aiguille après la fin de l'injection.

A cet effet, l'invention a pour objet une seringue d'injection selon la revendication 1.

Suivant des modes particuliers de réalisation, l'invention peut présenter l'une ou plusieurs des caractéristiques des revendications dépendantes.

L'invention sera mieux comprise à la lecture de la description qui va suivre, donnée uniquement à titre d'exemple et faite en se référant aux dessins sur lesquels :
- la figure 1 est une vue en coupe longitudinale d'une seringue non objet du brevet, avant utilisation ;
- la figure 2 est une vue en perspective à plus grande échelle du protecteur d'aiguille de la seringue de la figure 1 ;
- la figure 3 est une vue en coupe longitudinale de la seringue de la figure 1 représentée lors de l'injection du fluide dans le corps d'un patient ;
- la figure 4 est une vue en coupe longitudinale de la même seringue immédiatement après le retrait de l'aiguille du corps du patient ;
- la figure 5 est une vue en coupe longitudinale d'une variante de réalisation d'une seringue selon l'invention, avant utilisation ;
- la figure 6 est une vue en coupe longitudinale de la seringue de la figure 5 décalée angulairement de 90°, immédiatement avant l'injection du fluide dans le corps d'un patient ;
- la figure 7 est une vue en coupe longitudinale de la même seringue en fin d'injection : et
- la figure 8 est une vue en coupe longitudinale de la même seringue après le retrait de l'aiguille du corps du patient.

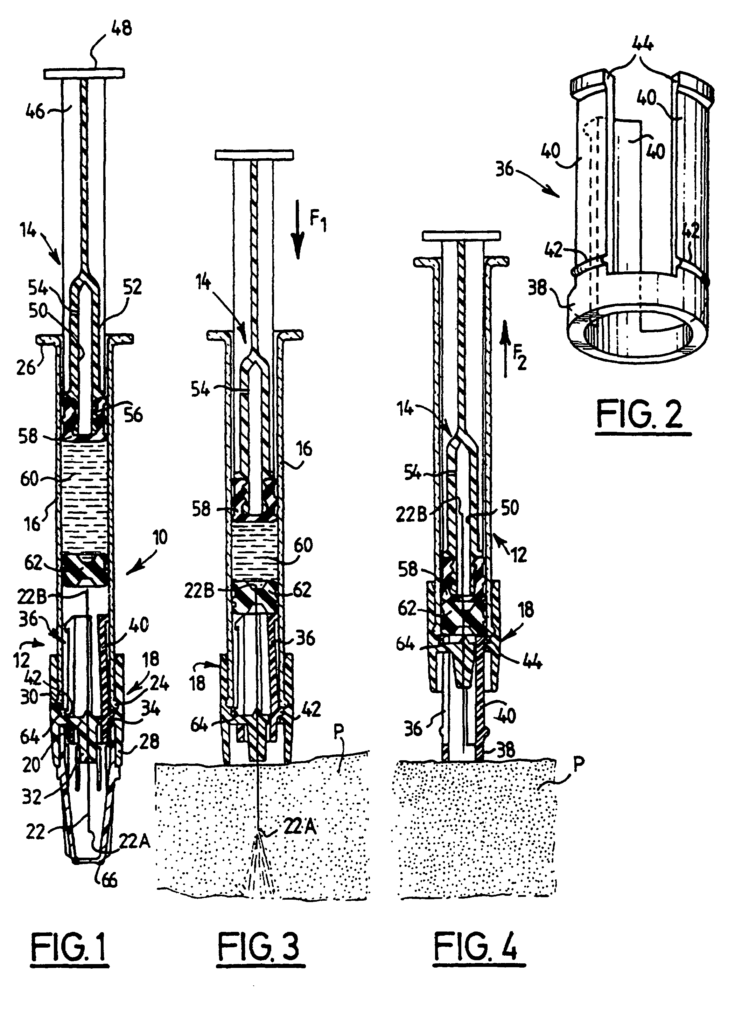

La seringue d'injection 10 représentée sur la figure 1 n'est pas l'objet du brevet. Cette seringue de forme générale de révolution d'axe X-X, est une seringue à usage unique, proposée prête à l'emploi et contenant déjà un fluide médical à injecter. Elle comporte essentiellement un corps de seringue 12 allongé et un piston arrière 14 d'actionnement monté déplaçable à l'intérieur du corps 12.

Le corps de seringue 12 est formé d'un tube 16 à l'extrémité avant duquel est fixé un porte-aiguille 18 portant une paroi avant 20 du corps de seringue. Cette paroi avant 20 est munie d'une aiguille d'injection traversante 22, dont une extrémité arrière 22B fait saillie à l'intérieur du corps 12.

Le tube 16 est réalisé par exemple en verre et a une section circulaire. Son extrémité avant est munie extérieurement d'un bourrelet périphérique 24 de maintien du porte-aiguille 18. Son extrémité arrière présente un rebord périphérique extérieur 26 facilitant la préhension du corps de seringue entre l'index et le majeur.

Le porte-aiguille 18 est délimité extérieurement par un manchon 28. La paroi avant 20 est venue de matière avec le manchon 28 et s'étend transversalement en un emplacement intermédiaire de celui-ci. Sur la paroi intérieure du manchon 28 est ménagée, légèrement en arrière de la paroi avant 20, une gorge périphérique 30 de réception du bourrelet 24.

La paroi avant 20 présente un plot axial 32 venu de matière, de fixation de l'aiguille d'injection 22. Ce plot est dirigé vers l'extrémité d'injection 22A de l'aiguille 22 et est reçu à l'intérieur de l'espace délimité par le manchon 28.

Trois ouvertures 34 identiques sont ménagées au travers de la paroi avant 20. Elles sont régulièrement réparties angulairement autour du plot 32 sur un même contour circulaire, et présentent une forme arquée.

Ces ouvertures 34 assurent le passage et le guidage d'un protecteur d'aiguille 36 représenté plus en détail sur la figure 2. Il présente à l'avant une bague de protection 38 en matière plastique rigide, dont les diamètres intérieur et extérieur sont adaptés pour que la bague 38 puisse se loger dans l'espace annulaire défini entre le plot 32 et le manchon 28. Cette bague est prolongée par trois jambages 40 identiques élastiquement déformables et espacés angulairement de 120°. Ces jambages 40 présentent en section une légère courbure correspondant à celle de la bague 38 et ont une longueur légèrement inférieure à la longueur de la partie de l'aiguille 22 reçue dans le corps de seringue 12.

Par ailleurs, chaque jambage 40 comporte extérieurement sur toute sa largeur un premier bombage 42, disposé légèrement en arrière de la bague 38, et un second bombage 44 disposé à son extrémité libre.

Le piston arrière 14 comporte un poussoir allongé 46 ayant une section en croix et présente à son extrémité arrière une pastille 48 d'appui du pouce de l'opérateur. A son extrémité opposée est prévu axialement un logement 50 ouvert sur l'avant, servant à la réception de l'extrémité arrière 22B ou proximale de l'aiguille 22 en fin d'injection. Ce logement 50, de forme allongée suivant l'axe X-X, a une section circulaire. Il est délimité par une paroi cylindrique 52 munie d'un évent 54 calibré.

La paroi 52 présente extérieurement à son extrémité avant trois bourrelets annulaires successifs 56 de fixation par emboîtement d'une membrane d'extrémité 58 en forme de cuvette. Cette membrane obture l'ouverture principale avant du logement 50, et est adaptée pour coulisser de manière étanche à l'intérieur du tube 16.

Tel que représenté sur la figure 1, le fluide à injecter 60 est disposé à l'intérieur du tube 16 dans un espace délimité par un piston avant 62 et la membrane 58 du piston arrière 14. Le piston avant 62 est formé par une cloison transversale perforable, entourée par une paroi latérale cylindrique munie de nervures périphériques afin d'assurer l'étanchéité au liquide et au gaz entre celle-ci et la paroi latérale intérieure du tube 16.

L'extrémité avant du tube 16 est emmanchée dans le manchon 28 et y est fixée par exemple par collage. La gorge périphérique 30 étant prévue légèrement en retrait par rapport à la paroi transversale 20, cette dernière délimite avec l'extrémité avant du tube 16 un canal annulaire 64, disposé immédiatement en arrière de la paroi 20 et dont le fond est formé par le manchon 28.

Le piston 62 est positionné légèrement en arrière de l'extrémité proximale 22B de l'aiguille d'injection.

Le protecteur 36 est monté au travers de la paroi avant 20, de telle sorte que la bague de protection avant 38 entoure le plot 32 et que les jambages 40 engagés dans les ouvertures respectives 34 s'étendent à l'intérieur du corps de seringue 12. Dans la position représentée à la figure 1, correspondant à la position escamotée du protecteur 36, les bombages 42 sont reçus à l'intérieur du canal 64, maintenant ainsi provisoirement le protecteur dans sa position escamotée.

Par ailleurs, un capuchon de protection 66 de l'aiguille 22 est emmanché à l'intérieur du manchon 28 et recouvre l'extrémité d'injection 22A de l'aiguille.

Afin de procéder à l'injection, l'opérateur retire le capuchon 66 puis introduit l'extrémité d'injection 22A de l'aiguille dans les tissus d'un patient P, tel que cela est représenté sur la figure 3. De manière classique, l'opérateur exerce alors avec le pouce une poussée sur le piston arrière 14 suivant le sens de la flèche F1, en prenant appui sous le rebord 26 avec l'index et le majeur.

La pression ainsi exercée, transmise par l'intermédiaire du liquide 60 au piston avant 62, provoque le déplacement de celui-ci vers l'extrémité proximale 22B de l'aiguille et sa perforation. Le déplacement du piston avant 62 empalé sur l'aiguille est stoppé lorsque celui-ci entre en contact avec les extrémités arrière des jambages 40 du protecteur, comme cela est représenté sur la figure 3.

A cet effet, les bombages 42 et le canal 64, formant des moyens d'encliquetage, sont dimensionnés de telle sorte que le protecteur 36 soit maintenu dans sa position rétractée malgré la pression exercée par le piston avant. Le fluide 60 s'écoule alors sous l'effet de la poussée du piston arrière 14 au travers de l'aiguille 22. Lorsque l'essentiel du fluide 60 est injecté, la membrane 58 vient au contact de la surface arrière du piston avant 62. La pression continue exercée par l'opérateur sur le piston arrière provoque le dégagement des bombages 42 du canal 64 par déformation élastique des jambages 40. Il s'ensuit le déplacement vers l'avant du protecteur 36. Lorsque la bague 38 entre en contact avec la peau du patient, la course du protecteur est stoppée et la poursuite du rapprochement du pouce appuyé sur la pastille 48 et de l'index et du majeur maintenus contre le rebord 26 provoque la remontée du corps de seringue 12 dans le sens de la flèche F2.

Comme cela est représenté sur la figure 4, on comprend que la remontée du corps 12 provoque l'extraction de l'aiguille 22 d'injection du corps P du patient. Par ailleurs, l'extrémité arrière 22B de l'aiguille transperce la membrane 58 et est reçue dans le logement 50.

La remontée du corps de seringue 12 se prolonge jusqu'à ce que les bombages d'extrémité 44 s'encliquettent dans le canal annulaire 64. Le protecteur 36 est alors en position active de protection et s'étend autour de l'aiguille 22. La face avant de la bague de protection 38 est alors située légèrement en avant de l'extrémité 22A de l'aiguille d'injection, et la bague 38 entoure cette extrémité, interdisant tout contact de celle-ci par un élément extérieur et évitant ainsi tout risque de piqûre contaminante pour l'opérateur. Par ailleurs, les bombages 44 reçus dans le canal 64 maintiennent le protecteur 36 solidement en position de protection, interdisant ainsi tout escamotage accidentel.

La membrane 58 ayant été perforée par l'extrémité arrière 22B de l'aiguille, toute réutilisation ultérieure de la seringue est rendue impossible. En effet, cette perforation interdit l'effet d'aspiration obtenu normalement à l'intérieur du corps lors du recul du piston arrière, du fait de la présence de l'évent 54. De même, le piston arrière perforé ne permet pas l'expulsion par l'aiguille d'injection 22 d'un éventuel liquide réintroduit dans le corps de seringue .

A cet effet, l'évent 54 est dimensionné de telle sorte que de l'air puisse circuler au travers de celui-ci lorsqu'il existe une différence de pression entre le logement 50 et le milieu ambiant, due par exemple au déplacement du piston arrière, mais qu'aucune traversée de liquide n'ait lieu pour une faible différence de pression. Ainsi, en fin d'injection, alors que l'extrémité 22A de l'aiguille est toujours introduite dans les tissus du patient, un éventuel reflux sanguin à travers l'aiguille 22 sous l'effet de la pression artérielle ne permet pas au sang de sortir du logement 50 à travers l'évent 54.

La seringue décrite ici est une seringue emplie lors de sa fabrication et proposée prête à l'emploi. Cependant, l'usage d'un protecteur ainsi que d'un piston arrière muni d'une membrane perforable est possible pour une seringue proposée vide et destinée à être emplie avant de procéder à l'injection. Dans ce cas, la seringue est proposée avec le piston avant 62 perforé, engagé sur l'aiguille 22 et en contact contre l'extrémité arrière des jambages 40 du protecteur. Par ailleurs, le piston arrière 14 est disposé de telle sorte que la membrane 58 soit immédiatement en arrière de l'extrémité proximale 22B de l'aiguille.

Dans ces conditions, il est possible, en tirant le piston arrière 14, d'aspirer le liquide à injecter au travers de l'aiguille 22. L'injection du liquide dans le corps du patient reprend les mêmes étapes que celles décrites précédemment, à l'exception de la première consistant à perforer le piston avant 62.

En variante, pour une telle seringue proposée vide, le piston avant 62 peut être supprimé. L'étanchéité est alors assurée au niveau des passages des jambages 40 dans les ouvertures 34 par un ajustement précis ou par une membrane souple recouvrant le protecteur.

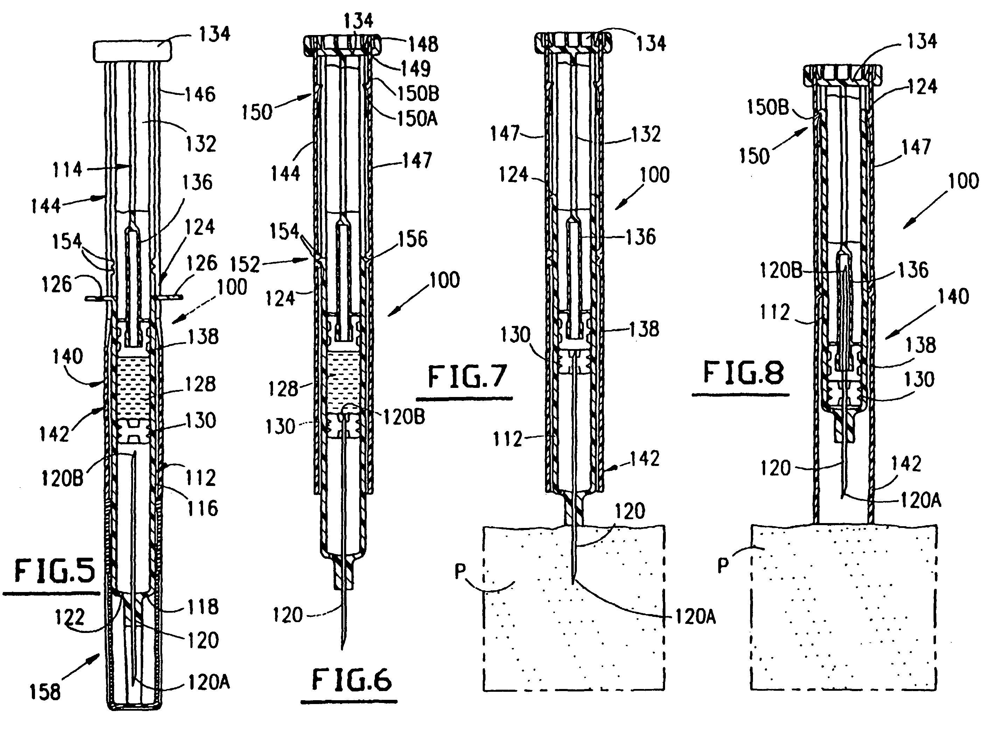

Sur les figures 5 à 8 est représentée une variante de réalisation d'une seringue d'injection selon l'invention.

La seringue d'injection représentée, portant la référence générale 100, est comme précédemment une seringue à usage unique, proposée prête à l'emploi et contenant déjà un fluide médical à injecter. Elle comporte essentiellement un corps de seringue 112 et un piston d'actionnement 114 monté déplaçable à l'intérieur du corps 112.

Le corps de seringue 112, réalisé par exemple en matière plastique, est formé d'un tube 116 obturé à une extrémité par une paroi avant 118 venue de matière. La paroi avant est traversée axialement par une aiguille d'injection 120 dont une extrémité avant 120A fait saillie à l'extérieur du corps. L'autre extrémité 120B fait saillie à l'intérieur du corps. Un évent 122 traverse la paroi avant 118.

L'extrémité arrière du tube 116 est ouverte et est bordée latéralement par un rebord périphérique 124 comportant deux oreilles radiales 126 diamétralement opposée formant des surfaces d'appui pour les doigts de l'opérateur lors de l'actionnement de la seringue.

Le corps de seringue 112 contient un fluide à injecter 128, maintenu entre un piston avant perforable 130 et le piston arrière d'actionnement 114.

Ce dernier comporte, comme dans le mode de réalisation précédent, un poussoir 132 de structure sensiblement analogue au poussoir 46. Ainsi, il comporte une pastille d'actionnement 134 à son extrémité arrière et à son extrémité avant un logement 136 muni d'un évent calibré. Le logement 136 ouvert sur l'avant est obturé par une membrane 138.

Le poussoir 132 est solidaire d'un protecteur d'aiguille mobile 140 monté déplaçable extérieurement le long du corps 112. Le protecteur d'aiguille 140 présente une forme essentiellement tubulaire. Il comporte à son extrémité avant un tronçon cylindrique 142 formant un fourreau. Ce dernier est prolongé à l'arrière par des moyens 144 de liaison au poussoir 132. Ces moyens de liaison comportent un tronçon tubulaire (figure 6) qui est venu de matière avec le tronçon avant 142 et qui est entaillé longitudinalement par deux fentes opposées 146 ouvertes à l'arrière et au travers desquelles font saillie les oreilles 126. Les fentes 146 délimitent ainsi entre elles sur le tronçon tubulaire 144 deux jambes de liaison 147 de section courbe.

A leur extrémité libre, les deux jambes de liaison 147 comportent des moyens d'encliquetage 148 au travers de lumières 149 de la pastille 134. Après encliquetage, l'extrémité arrière du protecteur 140 est collée ou soudée par ultrasons à la pastille 134.

Les jambes de liaison 147 comportent intérieurement en avant de la pastille 134 des moyens d'encliquetage 150 adaptés pour coopérer avec le rebord périphérique extérieur 124 ménagé à l'extrémité du corps de seringue. Ces moyens d'encliquetage 150 comportent par exemple une rampe 150A de hauteur progressivement croissante d'avant en arrière suivie par un front 150B.

En outre, les jambes de liaison 147 comportent, en avant des moyens d'encliquetage 150, des moyens 152 de maintien provisoire du protecteur d'aiguille dans une position escamotée sur le corps de seringue. Ces moyens 152 comportent par exemple deux bossages annulaires 154 ménagés intérieurement sur les jambes de liaison 147 et délimitant entre eux une gorge 156 de réception du rebord périphérique 124, comme représenté sur la figure 6.

Un capuchon de protection amovible 158 est disposé à l'extrémité avant de la seringue pour recouvrir l'extrémité 120A de l'aiguille.

Afin d'assurer le montage d'une telle seringue, on fabrique d'abord le corps de seringue 112 dans lequel est intégrée l'aiguille 120. On dispose ensuite dans le corps de seringue le piston avant 130, le liquide 128 et le piston arrière 114, préalablement équipé de la membrane 138. Le corps de seringue 112 est alors introduit par l'arrière dans le protecteur d'aiguille 140, les oreilles radiales 126 étant disposées dans les fentes 146. Lors de ce montage, le rebord 124 est engagé au-delà des moyens d'encliquetage 150 avec lesquels il coopère normalement. Ceci est rendu possible par la déformation élastique des jambes 147 dont l'extrémité arrière est libre. Les extrémités arrière des jambes de liaison 147 sont ensuite encliquetées dans les lumières 149 de la pastille 134, où elles sont enfin collées ou soudées par ultrasons afin d'assurer la fixation définitive du piston 114 et du protecteur d'aiguille 140. Le capuchon 158 est enfin mis en place pour recouvrir l'aiguille.

Dans la position représentée sur la figure 5, correspondant à l'état de livraison de la seringue, le piston avant 130 est écarté en arrière de l'extrémité 120B de l'aiguille. Ainsi, le fluide 128 n'est pas en contact avec l'aiguille. Le protecteur d'aiguille 140 est alors escamoté sur le corps de la seringue, le fourreau de protection 142 recouvrant le tube 116.

Afin d'utiliser la seringue, après retrait du capuchon 158, le piston 114 est légèrement enfoncé dans le corps de seringue, de sorte que le piston avant 130 s'empale sur l'extrémité 120B de l'aiguille, comme représenté sur la figure 6. Avant que le fluide 128 ne s'écoule au travers de l'aiguille 120, le rebord 124 s'encliquette dans la gorge 156, assurant ainsi un maintien provisoire du protecteur d'aiguille sur le corps de seringue, avant l'injection. L'extrémité de l'aiguille 120A est alors exposée, le fourreau de protection 142 étant entièrement disposé en arrière sur le corps 112.

Après introduction de l'extrémité 120A de l'aiguille dans le corps du patient, comme représenté sur la figure 7, le piston 114 est enfoncé progressivement dans le corps de seringue 112, ce qui provoque l'injection du fluide 128 par rapprochement du piston 114 du piston avant 130. Au cours de l'enfoncement du piston, le protecteur d'aiguille 140 se déplace axialement vers l'avant le long du corps de seringue jusqu'à la position représentée sur la figure 7 où le fluide 128 est totalement injecté.

Après injection totale du fluide 128, la poursuite de l'enfoncement du piston dans le corps provoque la mise en contact de l'extrémité avant du protecteur d'aiguille 140 avec la surface de la peau du patient. Une fois l'extrémité du protecteur en appui sur celle-ci, le coulissement continu du corps par rapport au protecteur d'aiguille provoque la remontée du corps de seringue 112 à l'intérieur du protecteur 140. Pendant cette phase, le piston 130 se déplace vers la paroi avant 118, l'air contenu dans la chambre délimitée entre le piston 130 et cette paroi 118 s'échappant par l'évent 122.

Comme cela est représenté sur la figure 8, en fin de remontée du corps de seringue, le protecteur d'aiguille 140 est en position active de protection. Dans cette position, le fourreau de protection 142 se trouve en avant de l'extrémité d'injection 120A de l'aiguille. Il entoure complètement celle-ci évitant ainsi tout risque de piqûre accidentel.

En outre, le rebord 124 s'encliquette en arrière du front 150B des moyens d'encliquetage 150, garantissant la retenue définitive du corps de seringue 112 et de l'aiguille 120 à l'intérieur du protecteur d'aiguille 140.

## Revendications

1. Seringue d'injection (100), du type comportant d'une part un corps de seringue (112) allongé comprenant un tube (116) et une paroi avant (118) munie d'une aiguille d'injection (120), et d'autre part un piston arrière d'actionnement (114) monté déplaçable á l'intérieur du tube (116), laquelle seringue est munie d'un protecteur d'aiguille mobile (140), déplaçable par rapport au corps (112) sous l'effet de l'enfoncement du piston d'actionnement (114) dans le corps (112) et pendant cet actionnement, entre une position escamotée en retrait de l'extrémité d'injection (120A) de l'aiguille (120) et une position active de protection dans laquelle l'extrémité avant du protecteur se trouve en avant de l'extrémité d'injection (120A) de ladite aiguille (120), le protecteur d'aiguille mobile (140) étant monté coulissant à l'extérieur du corps de seringue (112) et comportant d'une part un fourreau d'extrémité (142) de protection de l'aiguille (120) et d'autre part des moyens (144) de liaison dudit fourreau d'extrémité (142) au piston d'actionnement (114), lesquels moyens de liaison (144) s'étendent extérieurement le long du corps de seringue (112) jusqu'à l'extrémité du piston (114) faisant saillie par rapport au corps (112), **caractérisée en ce que** le protecteur d'aiguille mobile (140) est fixé au piston d'actionnement (114).

2. Seringue d'injection (100) selon la revendication 1, **caractérisée en ce que** le protecteur d'aiguille mobile (140) et le corps de seringue (112) comportent des moyens complémentaires (152) de maintien provisoire du protecteur (140) dans sa position escamotée.

3. Seringue d'injection (100) selon la revendication 1 ou 2, **caractérisée en ce que** le protecteur d'aiguille mobile (140) est essentiellement tubulaire et comporte des fentes longitudinales (146), le corps de seringue (112) comportant à son extrémité ouverte des oreilles radiales d'actionnement (126) qui font saillie par rapport audit protecteur d'aiguille mobile (140) au travers desdites fentes (146).

4. Seringue d'injection (100) selon l'une quelconque des revendications précédentes, **caractérisée en ce que** le protecteur (140) et le corps (112) comportent des moyens d'encliquetage complémentaires (150) du protecteur (140) sur le corps (112) dans sa position active de protection.

5. Seringue d'injection (100) selon l'une quelconque des revendications précédentes, dans laquelle l'aiguille d'injection (120) fait saillie à l'intérieur du corps de seringue (112), **caractérisée en ce que** le piston arrière (114) est muni en regard de l'extrémité proximale (120B) de l'aiguille (120) d'une membrane d'étanchéité (138) perforable par ladite extrémité proximale (120B) de l'aiguille (120) en fin d'enfoncement du piston (114) dans le corps (112).

6. Seringue d'injection (100) selon la revendication 5, **caractérisée en ce que** le piston arrière (114) est relié à une tige d'actionnement (132) comportant un logement (136) axial de réception de l'extrémité proximale (120B) de l'aiguille (120), disposé en arrière de ladite membrane (138).

7. Seringue d'injection (100) selon la revendication 6, **caractérisée en ce que** ledit logement (136) est délimité par une paroi pleine munie d'un ou plusieurs évents calibrés de communication avec le milieu ambiant.

## Patentansprüche

1. Injektionsspritze (100), von der Art, die einerseits einen länglichen Spritzenkörper (112) umfasst, der eine Röhre (116) und eine Vorderwand (118) umfasst, die mit einer Injektionsnadel (120) ausgestattet ist, und die andererseits einen hinteren Betätigungskolben (114) umfasst, der verschiebbar im Inneren der Röhre (116) angebracht, ist, wobei die Spritze mit einem beweglichen Nadelschutz (140) ausgestattet ist, der unter der Wirkung des Einschiebens des Betätigungskolbens (114) in den Körper (112) bezüglich des Körpers (112) verschieblich ist und, während dieser Betätigung, zwischen einer eingeschobenen, hinter das Injektionsende (120A) der Nadel (120) zurückgezogenen Position und einer aktiven Schutzposition verschiebbar ist, in welcher sich das vordere Ende des Schutzes vor dem Injektionsende (120A) der Nadel (120) befindet, wobei der bewegliche Nadelschutz (140) gleitend an der Außenseite des Spritzenkörpers (112) angebracht ist und einerseits eine Endschutzhülse (142) der Nadel (120) und andererseits Verbindungsmittel (144) der Endhülse (142) mit dem Betätigungskolben (114) umfasst, wobei sich die Verbindungsmittel (144) an der Außenseite entlang des Spritzenkörpers (112) bis zu dem Kolbenende (114) erstrecken, das bezüglich des Körpers (112) hervorsteht, **dadurch gekennzeichnet, dass** der bewegliche Nadelschutz (140) an dem Betätigungskolben (114) befestigt ist.

2. Injektionsspritze (100) nach Anspruch 1, **dadurch gekennzeichnet, dass** der bewegliche Nadelschutz (140) und der Spritzenkörper (112) komplementäre Mittel (152) umfassen, um den Schutz (140) provisorisch in seiner eingeschobenen Position zu halten.

3. Injektionsspritze (100) nach Anspruch 1 oder 2, **dadurch gekennzeichnet, dass** der bewegliche Nadelschutz (140) im Wesentlichen röhrenförmig ist und längliche Schlitze (146) umfasst, wobei der Spritzenkörper (112) an seinem offenen Ende radiale Betätigungsansätze (126) umfasst, die im Verhältnis zu dem beweglichen Nadelschutz (140) durch die Schlitze (146) hindurch hervorstehen.

4. Injektionsspritze (100) nach einem der vorhergehenden Ansprüche, **dadurch gekennzeichnet, dass** der Schutz (140) und der Körper (112) komplementäre Mittel (150) zum Einrasten des Schutzes (140) an dem Körper (112) in seiner aktiven Schutzposition umfassen.

5. Injektionsspritze (100) nach einem der vorhergehenden Ansprüche, bei welcher die Injektionsnadel (120) in das Innere des Spritzenkörpers (112) hineinragt, **dadurch gekennzeichnet, dass** der hintere Kolben (114) dem proximalen Ende (120B) der Nadel (120) gegenüberliegend mit einer Dichtungsmembran (138) ausgestattet ist, die durch das proximale Ende (120B) der Nadel (120) am Ende des Einschiebens des Kolbens (114) in den Körper (112) perforierbar ist.

6. Injektionsspritze (100) nach Anspruch 5, **dadurch gekennzeichnet, dass** der hintere Kolben (114) mit einem Betätigungsschaft (132) verbunden ist, der eine hinter der Membran (138) angeordnete axiale Aufnahme (136) zur Aufnahme des proximalen Endes (120B) der Nadel (120) umfasst.

7. Injektionsspritze (100) nach Anspruch 6, **dadurch gekennzeichnet, dass** die Aufnahme (136) durch eine durchgehende massive Wand begrenzt wird, die mit einer oder mehreren kalibrierten Entlüftungen zur Verbindung mit dem Umgebungsmilieu ausgestattet ist.

## Claims

1. Injection syringe (100), of the type comprising on the one hand an elongate syringe body (112) comprising a tube (116) and a front wall (118) provided with an injection needle (120) and on the other hand a rear actuating piston (114) movable within the tube (116), said syringe being provided with a movable needle guard (140), which is movable relative to the body (112) under the effect of the insertion of the actuating piston (114) in the body (112) and during this actuation, between a retracted position in which it is behind the injection end (120A) of the needle (120) and an active guarding position in which the front end of the guard is in front of the injection end (120A) of said needle (120), the movable needle guard (140) being mounted to slide outside the syringe body (112) and comprising on the one hand an end sheath (142) for protecting the needle (120) and on the other hand means (144) for connecting said end sheath (142) to the actuating piston (114), said connecting means (144) extending externally along the syringe body (112) to the end of the piston (114) projecting relative to the body (112), **characterised in that** the movable needle guard (140) is fixed to the actuating piston (114).

2. Injection syringe (100) according to claim 1, **characterised in that** the movable needle guard (140) and the syringe body (112) comprise complementary means (152) for temporarily holding the guard (140) in its retracted position.

3. Injection syringe (100) according to claim 1 or 2, **characterised in that** the movable needle guard (140) is substantially tubular and comprises longitudinal slots (146), the syringe body (112) comprising at its open end radial actuating lugs (126) which project relative to said movable needle guard (140) through said slots (146).

4. Injection syringe (100) according to anyone of the preceding claims, **characterised in that** the guard (140) and body (112) comprise complementary latching means (150) for latching the guard (140) onto the body (112) in its active guarding position.

5. Injection syringe (100) according to any one of the preceding claims, wherein the injection needle (120) projects inside the syringe body (112), **characterised in that** the rear piston (114) is provided, opposite the proximal end (120B) of the needle (120) with a sealing membrane (138) adapted to be perforated by said proximal end (120B) of the needle (120) when the piston (114) is fully inserted in the body (112).

6. Injection syringe (100) according to claim 5, **characterised in that** the rear piston (114) is connected to an actuating rod (132) comprising an axial recess (136) for accommodating the proximal end (120B) of the needle (120), arranged behind said membrane (138).

7. Injection syringe (100) according to claim 6, **characterised in that** said recess (136) is delimited by a solid wall provided with one or more calibrated vents for communicating with the ambient medium.
